Europäisches Patentamt

European Patent Office

Office européen des brevets

(19)

(11) Numéro de publication : **0 076 211**
**B1**

(12) **FASCICULE DE BREVET EUROPÉEN**

(45) Date de publication du fascicule du brevet :
27.05.87

(51) Int. Cl.⁴ : **G 01 N 33/92**

(21) Numéro de dépôt : **82401735.4**

(22) Date de dépôt : **27.09.82**

(54) Procédé et réactifs de séparation sélective des lipoprotéines de faible densité (LDL) et de quantification de leurs composants.

(30) Priorité : **28.09.81 FR 8118220**

(43) Date de publication de la demande :
**06.04.83 Bulletin 83/14**

(45) Mention de la délivrance du brevet :
**27.05.87 Bulletin 87/22**

(84) Etats contractants désignés :
**AT BE CH DE GB IT LI LU NL SE**

(56) Documents cités :
**EP-A- 0 006 822**
**EP-A- 0 009 596**
**WO-A-81 /011 99**
**US-A- 4 039 285**
**US-A- 4 215 993**
**CLINICAL CHEMISTRY, vol. 26, no. 13, décembre 1980, pages 1780-1786, Easton (USA);**
**CLINICAL CHEMISTRY, vol. 26, no. 13, décembre 1980, pages 1836-1838, Easton (USA);**
**CLINICAL CHEMISTRY, vol. 26, no. 13, décembre 1980, pages 1775-1779, Easton (USA);**
**CLINICAL CHEMISTRY, vol. 25, no. 4, avril 1979, pages 596-604, Easton (USA);**
**CLINICAL CHEMISTRY, vol. 27, no. 3, mars 1981, pages 371-374, Easton (USA);**
**CLINICAL CHEMISTRY, vol. 26, no. 9, août 1980, pages 1275-1277, Easton (USA);**

(73) Titulaire : **BIOMERIEUX, Société Anonyme dite Marcy l'Etoile**
**F-69260 Charbonnières-les-Bains (FR)**

(72) Inventeur : **Trouyez, Gérard**
**30, rue Chaziere**
**F-69004 Lyon (FR)**
Inventeur : **Alcindor, Louis-Gérald**
**22, rue du Sergent Bauchat**
**F-75012 Paris (FR)**

(74) Mandataire : **Gillard, Marie-Louise et al**
**Cabinet Beau de Loménie 55, Rue d'Amsterdam**
**F-75008 Paris (FR)**

## Description

La présente invention concerne la séparation et le dosage des lipoprotéines de faible densité (LDL) à l'aide d'un réactif précipitant adapté, et le dosage des composants qu'elles renferment.

La maladie athéromateuse, cérébrale ou cardiaque, représente un fléau des sociétés occidentales par les séquelles invalidantes et la mortalité qu'elle entraîne.

Les études, dans le domaine des facteurs favorisants, voire déclenchants, de cette maladie multiforme, ont montré l'importance de l'obésité, l'hypertension, le tabagisme et l'hyperlipidémie.

L'hyperlipidémie est caractérisée par l'augmentation de certaines classes de lipoprotéines, particules complexes associant des protéines spécifiques, ou apoprotéines du cholestérol libre ou estérifié des triglycérides et des phospholipides.

Le rôle des lipoprotéines est, dans l'organisme, de véhiculer les triglycérides, source d'énergie pour les cellules et le cholestérol, constituant important de leurs parois. En cas d'apport excessif de cholestérol, celui-ci peut s'accumuler et provoquer l'obstruction du réseau circulatoire.

Il est connu que l'augmentation du taux de cholestérol total n'est que médiocrement en corrélation avec le risque d'athérosclérose.

En effet, certains patients développent des signes précoces d'athérome, bien que leur cholestérol soit compris dans les limites de la normale (Rossner et al. 1978, 2, 577-579), alors que d'autres présentent un risque minime malgré un taux élevé de cholestérol (Avogaro et al. Clin. Chim. Acta, 1977, 77, 139-145).

Les acquisitions sur le métabolisme du cholestérol ont permis d'expliquer ces différences en montrant le rôle « épurateur » des HDL (ou lipoprotéines de haute densité) :

Schwartz et al. Science 1978, 200, 62-64 et le rôle athérogène des (LDL) ou lipoprotéines de faible densité.

Jenkins et al. Brit. Med. I, 1978, 298, 633-634.

Brown et al. Science 1976, 191, 150-154 ; les VLDL ou lipoprotéines de très faible densité et les chylomicrons étant peu ou pas athérogènes.

Ainsi, bien qu'il soit souhaitable de déterminer le taux de cholestérol lié aux LDL, il n'existe actuellement aucune technique faible, utilisable en routine et dont le coût abordable rende son utilisation possible, dans le domaine du dépistage et de la prévention du risque athéromateux.

Parmi les méthodes de dosage du cholestérol déjà proposées on peut citer celle décrite dans le brevet EP 6822, laquelle comprend la séparation des fractions lipoprotéines selon leur densité, puis la détermination de la teneur en cholestérol d'au moins l'une de ces fractions. Dans ce procédé la séparation est effectuée à partir d'un échantillon sérique, par précipitation sélective des fractions LDL et VLDL (renfermant l'apoprotéine B) au moyen d'une lectine capable de former des complexes avec des lipoprotéines.

La précipitation des chylomicrons, des lipoprotéines de très basse densité (VLDL) et basse densité (LDL) à l'aide de polyanions avec ou sans cations divalents a été précédemment décrite : Burstein M. et Scholnick M. R. et Morfin R. J. Lipi. Res. 1970, 11, 583 et permet le dosage du cholestérol lié aux lipoprotéines de haute densité (HDL). Les polyanions utilisés pour cette précipitation sont choisis parmi les suivants : héparine, polyéthylèneglycols, phosphotungstates, sulfate de dextrane et analogues.

La demanderesse a cherché à empêcher la précipitation des VLDL et chylomicrons et HDL, et à faire en sorte que les seules LDL soient précipitées.

Il a été découvert selon l'invention qu'il était possible de rendre le réactif précipitant connu, comprenant un ou des polyanions avec ou sans cations divalents, sélectif de la précipitation des LDL.

L'invention consiste donc en un procédé de précipitation sélective des lipoprotéines de faible densité dans les liquides biologiques ou les extraits tissulaires, sans précipiter, le cas échéant, les lipoprotéines de haute densité, par un réactif contenant un ou des polyanions tels que l'héparine, les polyéthylèneglycols, l'acide phosphotungstique, un sulfate de dextrane, associé(s) ou non à des cations divalents, caractérisé en ce que, pour éviter la précipitation concomitante des lipoprotéines de très basse densité et/ou des chylomicrons, on utilise un réactif contenant, en combinaison avec les éléments ci-dessus, un détergent anionique, cationique, ou non ionique, ledit détergent étant autre qu'un polyéthylèneglycol lorsque le réactif contenant un ou des polyanions est un polyéthylèneglycol.

Le poids moléculaire du ou des détergent(s) anionique(s), cationique(s) ou non ionique(s) adjoint(s) aux réactifs précipitant les VLDL, LDL et chylomicrons est de préférence supérieur à 200.

Comme détergent(s) on utilisera soit des polymères d'alcools ou d'amines bifonctionnels linéaires ou cycliques sur lesquels peuvent être greffés des alkyl ou aryl-éthers, soit des sels d'acides minéraux, tels que les polyphosphates ou des polysulfonates, soit des sels d'acides gras, soit des sels d'acides biliaires ou tout autre détergent ou agent tensio-actif adapté.

Parmi les polymères d'alcool utilisables comme détergents figurent les polyéthylèneglycols à longue chaîne tels que le polyéthylèneglycol 6000 et les éthers de polyéthylèneglycol tels que le Triton[R] X-100, ces détergents sont additionnés aux réactifs de précipitation à la concentration de 0,5 à 10 g/litre et de préférence 2 g/litre.

Le réactif de précipitation peut également contenir un polyéthylèneglycol qui joue le rôle de polyanion. Dans ce cas, sa concentration est beaucoup plus élevée, c'est-à-dire de 5 à 30 % (soit 50 à 300 g/litre de réactif) et on doit ajouter au réactif un détergent autre qu'un polyéthylèneglycol, par exemple un éther de polyéthylèneglycol tel que le Triton* X-100, ou des sels biliaires, à la concentration de 0,5 à 10 g par litre.

Selon une variante, les divers constituants des réactifs selon l'invention sont stabilisés de façon appropriée et permettent de provoquer la précipitation des LDL dans les liquides biologiques ou les extraits tissulaires.

Les réactifs obtenus ont été tout d'abord testés sur des lipoprotéines plasmatiques obtenues à partir, soit de sujets normaux, soit de sujets hospitalisés pour infarctus du myocarde, et séparées par ultracentrifugation préparative ; les LDL sont collectées entre les densités de 1,025 et 1,05, les HDL entre 1,063 et 1,21, les VLDL entre 1,006 et 1,019 selon les techniques habituelles.

La pureté de ces fractions a été confirmée par électrophorèse sur gel de polyacrylamide à gradient de concentration, le cholestérol est dosé par la technique de Allain, les phospholipides par la technique de Takayama, les triglycérides par la technique de Bucolo et David, les apoprotéines à l'aide d'antisérums spécifiques en immunoélectrophorèse, immunodiffusion radiale et immunoprécipitation.

La cinétique de la précipitation des LDL peut être également suivie par la mesure de l'augmentation de la turbidité à 620 nm.

Le précipité de LDL est dosé par des opérations consistant :

soit à apprécier la quantité de LDL précipitant par une mesure turbidimétrique ou néphélométrique,

soit à doser la quantité d'un des constituants des lipoprotéines précipitées, ce constituant pouvant être le cholestérol non estérifié, le cholestérol estérifié, le cholestérol total, les triglycérides, les phospholipides, les apoprotéines.

On a constaté que :

1) les fractions pures de LDL sont précipitées par le réactif selon l'invention décrit ci-dessus ;

2) les fractions de chylomicrons, VLDL et HDL ne précipitent pas et se retrouvent dans le surnageant ;

3) le sérum ou plasma humain ou animal, traité par ce réactif, donne un précipité ; seules les LDL sont retrouvées dans ce précipité ;

4) le précipité obtenu, solubilisé, dialysé et soumis à l'ultracentrifugation analytique, se comporte comme une fraction LDL ;

5) le précipité renferme de l'apoprotéine B, du cholestérol, des triglycérides et des phospholipides dans des proportions identiques à celles décrites pour les LDL ;

6) les taux de cholestérol, phospholipides, apoprotéines, obtenus avec le précipité, sont en corrélation satisfaisante avec les taux obtenus pour des lipoprotéines de faible densité séparées par ultracentrifugation, ceci tant pour les sérums d'individus normaux que pour des sérums de patients hospitalisés pour infarctus du myocarde, ou toute autre affection.

Il a été obtenu un défaut de précipitation pour des patients présentant des taux de triglycérides supérieurs à 7 mmol/l, mais ces individus ne font que rarement d'accident vasculaire, ainsi que l'ont souligné les différentes études épidémiologiques.

Le précipité de LDL, obtenu selon le procédé de l'invention, et séparé par décantation ou centrifugation lente peut être dissous par un agent chélatant tel que l'EDTA ou l'EGTA, l'acide iminodiacétique, les citrates et oxalates de sodium et de potassium et de préférence le citrate de sodium à une concentration de 1 à 100 mmol/l, et de préférence à 10 mmol/l.

Le précipité de LDL peut également être dissous par une combinaison d'un agent chélatant et d'un détergent.

Les constituants des LDL ainsi solubilisés, à savoir le cholestérol libre ou estérifié, les triglycérides, les phospholipides et apoprotéines peuvent alors être dosés selon les techniques habituelles.

Le réactif de solubilisation du précipité de LDL selon l'invention peut également permettre le dosage de ces constituants par l'un des procédés exposés ci-dessus.

Selon une variante, le réactif selon l'invention permet la solubilisation du précipité de LDL et le dosage du contenu de celui-ci en cholestérol. Un tel réactif est caractérisé par le fait que la solubilisation est due à la présence de chélatant (de préférence le citrate de sodium), associé à un détergent (de préférence le Triton* X-100) qui libère le contenu en cholestérol. Le cholestérol est ensuite oxydé par la cholestérol-oxydase, le peroxyde d'hydrogène formé étant mis en évidence par la réaction de Trinder, la présence ou l'absence de cholestérol estérase dans le réactif permettant de doser soit le cholestérol total, soit le cholestérol non estérifié du précipité de lipoprotéines de basse densité.

D'autre part, le dosage du cholestérol des LDL peut être pratiqué directement sur le culot de précipitation des LDL selon l'invention, à condition d'additionner au réactif utilisé pour le dosage enzymatique du cholestérol une quantité de 1 à 100 mmol/l, de préférence 10 mmol/l, de citrate de sodium.

Ainsi, les réactifs « cholestérol enzymatique PAP 100 » ou « cholestérol enzymatique PAP 250 » ainsi modifiés sont-ils utilisables pour le dosage direct du cholestérol des LDL contenus dans le culot.

Le dosage des phospholipides des LDL peut être également pratiqué directement sur le culot de précipitation, à condition de modifier le réactif « phospholipides enzymatiques 150 » de la même façon qu'indiqué ci-dessus pour le dosage du cholestérol.

On peut enfin envisager un système de réactifs composé d'un ou plusieurs réactifs de précipitation des lipoprotéines de faible densité et un ou plusieurs réactifs de dosage des lipoprotéines ainsi précipitées.

Les exemples suivants illustrent l'invention sans toutefois en limiter la portée.

Exemple 1

Le réactif précipitant les LDL consiste en une solution d'héparine dont la concentration est choisie entre 150 et 250 kilounités USP/1, de préférence 183 kilounités USP/1, associée au chlorure de manganèse, de 12 à 120 mmol/l, de préférence 92 mmol/l, et à un détergent qui est le polyéthylèneglycol 6 000 ou le Triton X-100 dont la concentration peut varier de 0,5 à 10 g/l, de préférence 2 g/l, ou un sel biliaire à la concentration de 0,5 à 5 g/l.

100 µl de sérum sont mis en contact pendant 5 à 60 min, de préférence 30 min, avec 1 ml de ce réactif précipitant. Le mélange est centrifugé pendant 15 min à 3 000 g, le précipité décanté est solubilisé par une solution de NaCl 0,15 M, additionné d'EDTA ou de citrate de sodium 0,15 M. Ce précipité resolubilisé peut être analysé par les techniques décrites plus haut. Il contient les LDL.

Exemple 2

La méthodologie est identique, mais le chlorure de manganèse est remplacé par le chlorure de calcium à la même concentration.
On obtient le même résultat.

Exemple 3

Le réactif précipitant est constitué par une solution de sulfate de dextrane 500, dont la concentration est choisie entre 10 et 200 g/l, de préférence 50 g/l, associé au chlorure de calcium à 2,5 g/l, les détergents utilisés étant identiques à ceux mentionnés dans l'exemple 1, dans les mêmes limites de concentration.
On obtient le même résultat.

Exemple 4

On utilise une solution de polyéthylèneglycol 6 000 dont la concentration est choisie entre 5 et 30 %, de préférence 15 %, associée à un sel biliaire dont la concentration est de 0,5 à 5 g/l, de préférence le taurodésoxycholate de sodium (1-3 g/l).

200 µl de ce réactif et 200 µl de sérum sont mis en contact 30 min, puis le mélange est centrifugé 5 min à 3 000 g. Le surnageant séparé est analysé. Le précipité contient les LDL.

Exemple 5

On utilise une solution de phosphotungstate de sodium dont la concentration est choisie entre 10 et 100 g/l, de préférence 40 g/l, et de chlorure de magnésium à 100 g/l.
Les sels biliaires ou les alcools polyéthoxylés ci-dessus sont ajoutés aux concentrations ci-dessus (respectivement 0,5 à 5 g/l et 0,5 à 10 g/l).

500 µl de sérum et 50 µl de réactif précipitant sont mélangés et incubés 10 min à température ambiante. Le mélange est centrifugé 15 min à 5 000 tr/min. Le précipité contient les LDL.

Exemple 6

On utilise une solution de sulfate de dextrane 500 dont la concentration est choisie entre 0,1 et 10 g/l, de préférence 0,4 g/l, associée au chlorure de calcium entre 0,1 et 20 mmol, de préférence 10 mmol/l, et au taurodésoxycholate choisi entre 0,1 et 10 g/l, de préférence 0,8 g/l.

50 µl de sérum sont mélangés à 500 µl de réactif précipitant, incubés pendant 15 à 60 min à température ambiante; le mélange est centrifugé 20 min à 5 000 g, le culot obtenu contient les LDL.

**Revendications**

1. Procédé de précipitation sélective des lipoprotéines de faible densité dans les liquides biologiques ou les extraits tissulaires, sans précipiter, le cas échéant, les lipoprotéines de haute densité, par un réactif contenant un ou des polyanions tels que l'héparine, les polyéthylèneglycols, l'acide phosphotungstique, un sulfate de dextrane, associé(s) ou non à des cations divalents, caractérisé en ce que, pour éviter la précipitation concomitante des lipoprotéines de très basse densité et/ou des chylomicrons, on utilise un réactif contenant, en combinaison avec les éléments ci-dessus, un détergent anionique, cationique, ou non ionique, ledit détergent étant autre qu'un polyéthylèneglycol lorsque le réactif contenant un ou des polyanions est un polyéthylèneglycol.

2. Procédé selon la revendication 1, caractérisé en ce que ledit détergent est de poids moléculaire supérieur à 200.

3. Procédé selon l'une quelconque des revendications 1 et 2, caractérisé en ce que l'on utilise comme détergent combiné soit des polymères d'alcool ou d'amines bifonctionnels linéaires ou cycliques sur lesquels peuvent être greffés des alkyl- ou aryléthers, soit des sels d'acides minéraux, tels que les polyphosphates ou des polysulfonates, soit des sels d'acides gras, soit des sels d'acides biliaires.

4. Procédé selon l'une des revendications 1 à 3, caractérisé en ce que le détergent est présent dans le réactif de précipitation à la concentration de 0,5 à 10 g/l.

5. Procédé de dosage des LDL, caractérisé en ce qu'on précipite les LDL par un procédé selon l'une quelconque des revendications 1 à 4, on sépare ce précipité, après quoi on effectue les opérations suivantes consistant :
soit à apprécier la quantité de LDL précipitant par une mesure turbidimétrique ou néphélométrique,
soit à doser la quantité d'un des constituants des lipoprotéines précipitées, ce constituant pouvant être le cholestérol non estérifié, le cholestérol estérifié, le cholestérol total, les triglycérides, les phospholipides, les apoprotéines.

6. Procédé de dosage des LDL selon la revendication 5, caractérisé en ce que le précipité obtenu par un procédé selon l'une quelconque des revendications 1 à 4 est d'abord dissous.

7. Procédé de dosage selon la revendication 6, caractérisé en ce que ladite dissolution du précipité met en jeu des agents chélatants, tels que les citrates et oxalates de Na et de K, l'EDTA, l'EGTA, l'acide iminodiacétique, associés ou non à des détergents.

8. Réactif de précipitation sélective des LDL à base d'un ou de polyanion(s) tels que l'héparine, les polyéthylèneglycols, l'acide phosphotungstique, un sulfate de dextrane, associé(s) ou non à des cations divalents, caractérisé en ce que, pour éviter la précipitation concomitante des lipoprotéines de très basse densité et/ou des chylomicrons, ce réactif contient également un détergent anionique, cationique ou non ionique.

9. Réactif selon la revendication 8, caractérisé en ce que ledit détergent est de poids moléculaire supérieur à 200.

10. Réactif selon l'une quelconque des revendications 8 et 9, caractérisé en ce que l'on utilise comme détergent combiné soit des polymères d'alcool ou d'amines bifonctionnels linéaires ou cycliques sur lesquels peuvent être greffés des alkyl- ou aryl-éthers, soit des sels d'acides minéraux, tels que les polyphosphates ou des polysulfonates, soit des sels d'acides gras, soit des sels d'acides biliaires.

11. Réactif selon l'une quelconque des revendications 8 à 10, caractérisé en ce que le détergent est présent à la concentration de 0,5 à 10 g/litre.

12. Réactif selon l'une quelconque des revendications 8 à 11, caractérisé en ce qu'il contient de l'héparine, ou un phosphotungstate, et un sel d'un cation bivalent, associés à un détergent choisi parmi les polyéthylèneglycols, les éthers de polyéthylèneglycols et les sels biliaires.

13. Réactif selon l'une quelconque des revendications 8 à 11, caractérisé en ce qu'il contient du polyéthylèneglycol 6 000 à la concentration de 50 à 300 g/l associé à un détergent choisi parmi les éthers de polyéthylèneglycol et les sels biliaires.

14. Réactif selon l'une quelconque des revendications 8 à 11, caractérisé en ce qu'il contient du sulfate de dextrane et des ions calcium associés à un sel biliaire, de préférence le taurodésoxycholate.

15. Réactif de dissolution du précipité de LDL obtenu par l'emploi du réactif selon l'une quelconque des revendications 8 à 14, caractérisé en ce qu'il contient au moins un agent chélatant, tel que les citrates et oxalates de Na et de K, l'EDTA, l'EGTA, l'acide iminodiacétique et de préférence le citrate de sodium, à la concentration de 1 à 100 mmol/litre.

16. Réactif selon la revendication 15, caractérisé en ce qu'il permet à la fois la solubilisation du précipité de LDL et le dosage de celui-ci selon les procédés indiqués dans la revendication 5.

17. Réactif selon la revendication 16, permettant à la fois la solubilisation du précipité de LDL et le dosage du contenu de celui-ci en cholestérol, caractérisé en ce qu'il contient un chélatant, de préférence le citrate de sodium, associé à un détergent, de préférence le Triton* X-100.

18. Procédé de dosage direct du cholestérol dans le précipité de LDL obtenu selon l'une des revendications 1 à 4, caractérisé en ce qu'on additionne au réactif utilisé pour le dosage enzymatique du cholestérol de 1 à 100 mmol/l et de préférence 10 mmol/l de citrate de sodium.

19. Procédé de dosage direct des phospholipides dans le précipité de LDL obtenu selon l'une des revendications 1 à 4, caractérisé en ce qu'on additionne au réactif utilisé pour le dosage enzymatique des phospholipides de 1 à 100 mmol/l, et de préférence 10 mmol/l de citrate de sodium.

20. Un système de réactifs composé d'un ou de plusieurs réactifs de précipitation des lipoprotéines de faible densité selon l'une quelconque des revendications 8 à 14, et un ou plusieurs réactifs de dosage des lipoprotéines ainsi précipitées.

**Claims**

1. Process for selectively precipitating low density lipoproteins in biological liquids or tissue extracts, without precipitating, as the case may be, high density lipoproteins, by a reagent comprising one or more polyanions such as heparin, polyethylene glycols, phosphotungstic acid, a dextrane sulphate, associated or not associated with divalent cations, characterized in that, in order to prevent the concomitant precipitation of very low density lipoproteins and/or of chylomicrons, a reagent comprising in combination with the above elements an anionic, a cationic or non ionic detergent is used, said detergent being other than a polyethylene glycol when the reagent containing one or more polyanions is a polyethylene glycol.

2. Process according to claim 1, characterized in that said detergent has a molecular weight higher than 200.

3. Process according to any one of claims 1 and 2, characterized in that it comprises using as combined detergent, either alcohol polymers, or linear or cyclic bifunctional amines on which may be grafted alkyl or arylethers, or salts of mineral acids, such as polyphosphates or polysulphonates, or fatty acids salts, or biliary acids salts.

4. Process according to any one of claims 1 to 3, characterized in that the detergent is present in the precipitating reagent at the concentration of 0.5 to 10 g/litre.

5. Process for determining LDL, characterized in that the LDL are precipitated by a process according to any one of claims 1 to 4, said precipitate is separated, the following operations are then carried out which consist in :

either determining the quantity of precipitating LDL by a turbidimetric or nephelometric measurement,

or in determining the quantity of one of the constituents of the precipitated lipoproteins, said constituent may be non esterified cholesterol, esterified cholesterol, total cholesterol, triglycerides, phospholipides, apoproteins.

6. Process for determining LDL according to claim 5, characterized in that the precipitate obtained by a process according to any one of claims 1 to 4 is first dissolved.

7. Process for determining LDL according to claim 6, characterized in that said dissolution of the precipitate uses chelating agents, such as Na and K citrates and oxalates, EDTA, EGTA, iminodiacetic acid, associated or not associated with detergents.

8. Reagent of selective precipitation of LDL containing one or more polyanions such as heparin, polyethylene glycols, phosphotungstic acid, a dextrane sulphate, associated or not associated with divalent cations, characterized in that, in order to avoid the concomitant precipitation of very low density lipoproteins and/or of chylomicrons, said reagent also contains an anionic, a cationic or non-ionic detergent.

9. Reagent according to claim 8, characterized in that said detergent has a molecular weight higher than 200.

10. Reagent according to any one of claims 8 and 9, characterized in that it comprises using as combined detergent, either alcohol polymers or linear or cyclic bifunctional amines, on which alkyl- or arylethers may be grafted, or salts of mineral acids, such as polyphosphates or polysulphonates or fatty acid salts, or salts of biliary acids.

11. Reagent according to any one of claims 8 to 10, characterized in that the detergent is present at the concentration of 0,5 to 10 g/litre.

12. Reagent according to any one of claims 8 to 11, characterized in that it contains heparin, a phosphotungstate, and a salt of bivalent cation, associated with a detergent selected from among polyethylene glycols, ethers of polyethylene glycols and the biliary salts.

13. Reagent according to any one of claims 8 to 11, characterized in that it contains polyethylene glycol 6 000 at the concentration of 50 to 300 g/litre associated with a detergent selected from among the ethers of polyethylene glycols and the biliary salts.

14. Reagent according to any one of claims 8 to 11, characterized in that it contains dextrane sulphate and calcium ions associated with a biliary salt, preferably taurodesoxycholate.

15. Reagent for dissolving precipitate of LDL obtained by the use of the reagent according to any one of claims 8 to 14, characterized in that it contains at least a chelating agent, such as Na and K citrates and oxalates, EDTA, EGTA, iminodiacetic acid and preferably sodium citrate, at the concentration of 1 to 100 mmol/litre.

16. Reagent according to claim 15, characterized in that is enables both the solubilization of the LDL precipitate and the proportioning thereof according to the processes indicated in claim 5.

17. Reagent according to claim 16 enabling both the solubilization of the precipitate of LDL and the proportioning of its cholesterol content, characterized in that it contains a chelatant, preferably sodium citrate associated with a detergent, preferably Triton* X-100.

18. Process for direct determination of cholesterol in the LDL precipitate obtained according to any one of claims 1 to 4, characterized in that to the reagent used for the enzymatic determination of cholesterol are added between 1 and 100 mmol/litre, and preferably 10 mmol/litre of sodium citrate.

19. Process for direct determination of phospholipides in the LDL precipitate obtained according to any one of claims 1 to 4, characterized in that to the reagent used for the enzymatic determination of phospholipides are added between 1 and 100 mmol/litre, and preferably 10 mmol/litre of sodium citrate.

20. A system of reagents comprising one or more reagents precipitating low density lipoproteins according to any one of claims 8 to 14, and one or more reagents determining the precipitated lipoproteins.

**Patentansprüche**

1. Verfahren zur selektiven Ausfällung von Lipoproteinen geringer Dichte in biologischen Flüssigkeiten oder Zellextrakten, gegebenenfalls ohne Ausfällen von Lipoproteinen hoher Dichte, mittels eines ein oder mehrere Polyanionen, wie Heparin, Polyäthylenglykole, Phosphorwolframsäure, Dextransulfat, gewünschtenfalls in Verbindung mit divalenten Kationen, enthaltenden Reagens, dadurch gekennzeichnet, daß man zur Vermeidung des gleichzeitigen Ausfällens von Lipoproteinen sehr geringer Dichte und/oder von Chylomikronen ein Reagens verwendet, das in Kombination mit den obengenannten Elementen ein anionisches, kationisches oder nichtionisches Detergens enthält, welches Detergens nicht Polyäthylenglykol ist, wenn das ein oder mehrere Polyanionen enthaltende Reagens Polyäthylenglykol ist.

2. Verfahren nach Anspruch 1, dadurch gekennzeichnet, daß das Detergens ein Molgewicht von mehr als 200 hat.

3. Verfahren nach einem der Ansprüche 1 und 2, dadurch gekennzeichnet, daß man als kombiniertes Detergens entweder lineare oder cyclische bifunktionelle Alkohol- oder Aminpolymerisate, auf denen Alkyl- oder Aryläther aufgepfropft sein können, oder Salze von Mineralsäuren, wie Polyphosphate oder Polysulfonate, oder Salze von Fettsäuren oder Gallensäuresalze verwendet.

4. Verfahren nach einem der Ansprüche 1 bis 3, dadurch gekennzeichnet, daß das Detergens im Fällungsreagens in einer Konzentration von 0,5 bis 10 g/l vorhanden ist.

5. Verfahren zur LDL-Bestimmung, dadurch gekennzeichnet, daß man die LDL mittels eines Verfahrens nach einem der Ansprüche 1 bis 4 ausfällt, diesen Niederschlag abtrennt, worauf man die folgenden Arbeitsgänge ausführt :

entweder man bestimmt die ausgefällte LDL-Menge mittels turbidimetrischer oder nephelometrischer Messung,

oder man bestimmt die Menge eines der Bestandteile der ausgefällten Lipoproteine, welcher Bestandteil nichtverestertes Cholesterin, verestertes Cholesterin, Gesamtcholesterin, Triglyceride, Phospholipide, Apoproteine sein kann.

6. Verfahren zur LDL-Bestimmung nach Anspruch 5, dadurch gekennzeichnet, daß der mittels eines Verfahrens nach einem der Ansprüche 1 bis 4 erhaltene Niederschlag zuerst gelöst wird.

7. Bestimmungsverfahren nach Anspruch 6, dadurch gekennzeichnet, daß das Auflösen des Niederschlags Chelatisierungsmittel, wie Na- und K-Citrate und -Oxalate, EDTA, EGTA, Iminodiessigsäure, gewünschtenfalls in Verbindung mit Detergentien, ins Spiel bringt.

8. LDL-selektives Fällungsreagens auf Basis eines oder mehrerer Polyanionen, wie Heparin, Polyäthylenglykole, Phosphorwolframsäure, Dextransulfat, gewünschtenfalls in Verbindung mit divalenten Kationen, dadurch gekennzeichnet, daß zur Vermeidung des gleichzeitigen Ausfällens von Lipoproteinen sehr niedriger Dichte und/oder von Chylomikronen dieses Reagens auch ein anionisches, kationisches oder nichtionisches Detergens enthält.

9. Reagens nach Anspruch 8, dadurch gekennzeichnet, daß das Detergens ein Molgewicht von mehr als 200 hat.

10. Reagens nach einem der Ansprüche 8 und 9, dadurch gekennzeichnet, daß man als kombiniertes Detergens entweder lineare oder cyclische bifunktionelle Alkohol- oder Aminpolymerisate, auf denen Alkyl- oder Aryläther aufgepfropft sein können, oder Salze von Mineralsäuren, wie Polyphosphate oder Polysulfonate, oder Salze von Fettsäuren oder Gallensäuresalze verwendet.

11. Reagens nach einem der Ansprüche 8 bis 10, dadurch gekennzeichnet, daß das Detergens in einer Konzentration von 0,5 bis 10 g/l vorhanden ist.

12. Reagens nach einem der Ansprüche 8 bis 11, dadurch gekennzeichnet, daß es Heparin oder ein Phosphorwolframat und ein Salz eines bivalenten Kations in Verbindung mit einem Detergens, ausgewählt aus Polyäthylenglykolen, Polyäthylenglykoläthern und Gallensäuresalzen, enthält.

13. Reagens nach einem der Ansprüche 8 bis 11, dadurch gekennzeichnet, daß es Polyäthylenglykol 6 000 in einer Konzentration von 50 bis 300 g/l in Verbindung mit einem Detergens, ausgewählt aus Polyäthylenglykoläthern und Gallensäuresalzen, enthält.

14. Reagens nach einem der Ansprüche 8 bis 11, dadurch gekennzeichnet, daß es Dextransulfat und Calciumionen in Verbindung mit einem Gallensäuresalz, vorzugsweise Taurodesoxycholat, enthält.

15. Lösungsreagens für den durch Anwendung des Reagens nach einem der Ansprüche 8 bis 14 erhaltene LDL-Niederschlag, dadurch gekennzeichnet, daß es mindestens ein Chelatisierungsmittel, wie Na- und K-Citrate oder -Oxalate, EDTA, EGTA, Iminodiessigsäure, vorzugsweise Natriumcitrat, in einer Konzentration von 1 bis 100 mMol/l enthält.

16. Reagens nach Anspruch 15, dadurch gekennzeichnet, daß es gleichzeitig die Auflösung des LDL-Niederschlags und die Bestimmung desselben nach den im Anspruch 5 aufgezeigten Verfahren ermöglicht.

17. Reagens nach Anspruch 16, das gleichzeitig das Auflösen des LDL-Niederschlags und die Bestimmung des Cholesteringehalts desselben ermöglicht, dadurch gekennzeichnet, daß es ein Chelatisierungsmittel, vorzugsweise Natriumcitrat, in Verbindung mit einem Detergens, vorzugsweise Triton® X-100, enthält.

18. Verfahren zur direkten Bestimmung von Cholesterin in dem nach einem der Ansprüche 1 bis 4 erhaltenen LDL-Niederschlag, dadurch gekennzeichnet, daß man zu dem zur enzymatischen Bestimmung von Cholesterin verwendeten Reagens 1 bis 100 mMol/l, vorzugsweise 10 mMol/l, Natriumcitrat zugibt.

19. Verfahren zur direkten Bestimmung von Phospholipiden in dem nach einem der Ansprüche 1 bis 4 erhaltenen LDL-Niederschlag, dadurch gekennzeichnet, daß man zu dem zur enzymatischen Bestimmung der Phospholipide verwendeten Reagens 1 bis 100 mMol/l, vorzugsweise 10 mMol/l, Natriumcitrat zugibt.

20. System von Reagenzien, gebildet durch ein oder mehrere Reagenzien zur Ausfällung von Lipoproteinen geringer Dichte nach einem der Ansprüche 8 bis 14 und einem oder mehreren Reagenzien zur Bestimmung der so ausgefällten Lipoproteine.